# EUROPEAN PATENT APPLICATION

(11) **EP 3 733 861 A1**
(43) Date of publication of application: **04.11.2020**
(21) Application number: 17935873.4
(22) Date of filing: 28.12.2017
(51) Int. Cl.: C12P 21/00

(54) **IN VITRO PROTEIN SYNTHESIS SYSTEM, KIT AND PREPARATION METHOD THEREFOR**

(71) Applicant: Kangma-Healthcode (Shanghai) Biotech Co., Ltd, Pudong New Area Shanghai 201321 (CN)
(72) Inventor: GUO, Min, Shanghai 201321 (CN); ZHOU, Zijian, Shanghai 201321 (CN); YU, Xue, Shanghai 201321 (CN)
(74) Representative: Cabinet Chaillot
(86) International application number: PCT/CN2017/119540
(87) International publication number: WO 2019/127259

(57) **Abstract**

An *in vitro* protein synthesis system, a kit and a preparation method therefor. The *in vitro* protein synthesis system is formed by mixing (a) cell extract and (b) first reaction promoter in a specific proportion, and the synthetic efficiency of a heterologous protein can be improved.

## Description

### BACKGROUND OF THE INVENTION

### 1. Technical Field

The present invention relates to the field of biotechnology, and more particularly, to an *in vitro* protein synthesis system, a kit thereof and a preparation method therefor.

### 2. Description of the Related Art

Biological reactions, i.e., biochemical reactions, refer to chemical reactions that take place in organisms. These reactions are catalyzed by enzymes. Enzymes and reactants should be dissolved in the water of the internal environment before the reaction begins. The water is provided as a carrier and a medium for *in vivo* substances^{[1]}. In the last few decades of the 20^{th} century, biochemistry has made great achievements in explaining life processes, and now almost all fields related to life sciences such as botany, medicine and genetics are engaged in biochemical research^{[2]}.

The traditional protein expression system refers to a multistep cascade biochemical reaction that expresses exogenous genes through model organisms such as bacteria, fungi, plant cells or animal cells ^{[3-4]}. With the development of science and technology, a cell-free expression system, also known as an *in vitro* protein synthesis system, came into being. In the cell-free expression system, exogenous target mRNA or DNA is used as template for protein synthesis, and substrates required for protein synthesis, protein factors related to transcription and translation and other substances are added and supplemented through manual control, so that synthesis of target proteins (herein equivalent to objective proteins) can be achieved ^{[5-7]}. Expression of proteins in an *in vitro* translation system does not require steps of plasmid construction, transformation, cell culture, cell collection and cell disruption. It is a fast, time-saving, and convenient way of protein expression ^{[8]}. With the development of science and technology, protein synthesis systems have begun to be widely used.

The biochemical reactions that take place in organisms or cells can be automatically regulated by positive and negative feedback in a complex network system, and all the biochemical reactions that take place in organisms are reactions away from the equilibrium point, which need to obtain energy from the outside or to output substances, energy and entropy to the outside ^{[9]}. In cells, the removal of by-products of biological reactions is mainly achieved by the body's metabolism, the maintenance of the body's homeostasis, the catalytic action of biological enzymes, and the redox reactions away from the equilibrium point in cells, but it is difficult to realise these biochemical reactions by using *in vitro* biotechnology^{[10]}. In addition, one shortcoming of the protein synthesis system is that the yield is not high, the reaction is not fast enough and sensitive enough. In the *in vitro* biosynthesis system, the by-products, such as free phosphate ion and pyrophosphate ion (PPi), of reactions such as *in vitro* protein synthesis, *in vitro* transcription, kinase reaction, ATPase reaction, ion pump reaction and others, will limit the yield of target products.

Currently, other methods are known to remove the by-products of reactions. For example, perform a semi-continuous reaction to control by-products such as free phosphate ion by using a method that allows passive diffusion of small molecules between the reaction mixture and the dialysate by using a semi-permeable membrane. However, there are defects that the cost of a semi-permeable membrane device for dialysis is high, the reaction system cannot be amplified, and the reaction products are liable to block the pores of the semi-permeable membrane.

Accordingly, there is an urgent need in the art to develop and establish a method capable of improving the efficiency of protein synthesis in the protein synthesis system through controlling the content of free phosphate ion in the protein synthesis system by *in situ* removal of free phosphate ion.

### SUMMARY OF THE INVENTION

One object of the present invention is to provide a method for improving the efficiency of protein synthesis in the protein synthesis system through controlling the content of free phosphate ion in the protein synthesis system by *in situ* removal of free phosphate ion.

According to the first aspect, the present invention provides an *in vitro* protein synthesis system, comprising:
(a) cells, cell extract, or a combination thereof;
(b) a first reaction promoter, and the first reaction promoter is selected from the group consisting of aluminum, aluminum salt, oxy-aluminum composite, and combinations thereof.

In another preferred embodiment, the cells are selected from the group consisting of prokaryotic cells and eukaryotic cells.

In another preferred embodiment, the cells are selected from the group consisting of *E. coli,* bacteria, mammalian cells (e.g., HF9 cells, Hela cells, CHO cells, HEK293 cells), plant cells, yeast cells, and combinations thereof.

In another preferred embodiment, the yeast cells are selected from the group consisting of *Saccharomyces cerevisiae* cells, *Pichia pastoris* cells, *Kluyveromyces* cells, and combinations thereof; preferably, the yeast cells include *Kluyveromyces* cells, and more preferably, the yeast cells are *Kluyveromyces lactis* cells.

In another preferred embodiment, the protein synthesis system further comprises:
(b') a second reaction promoter, wherein the second reaction promoter is selected from the group consisting of monosaccharide, disaccharide, oligosaccharide, soluble starch, and combinations thereof;
(c) polyethylene glycol;
(d) optional exogenous sucrose; and
(e) an optional solvent, wherein the solvent is water or an aqueous solvent.

In another preferred embodiment, the protein synthesis system includes yeast-based *in vitro* protein synthesis system (e.g., *Kluyveromyces*-based *in vitro* protein synthesis system, preferably, *Kluyveromyces lactis* based *in vitro* protein synthesis system).

In another preferred embodiment, the oxy-aluminum composite includes oxy-aluminum nanoparticles.

In another preferred embodiment, the oxy-aluminum composite includes Al₂O₃.

In another preferred embodiment, the oxy-aluminum composite is in a form of particle form or nanoform.

In another preferred embodiment, the particle size of the oxy-aluminum composite is in a range of 0.5-20 mm, preferably, in a range of 0.8-10 mm, more preferably, in a range of 1-5 mm.

In another preferred embodiment, the average weight of the oxy-aluminum composite is in a range of 1-80 mg, preferably, in a range of 3-50 mg, more preferably, in a range of 4-30 mg.

In another preferred embodiment, in the first reaction promotor, the concentration (v/v) of the oxy-aluminum composite is in a range of 0.5%-20%, preferably, in a range of 0.8%-10%, more preferably, in a range of 1%-10%, more preferably, in a range of 1%-5%, more preferably, in a range of 2%-3%, based on the total volume of the first reaction promoter.

In another preferred embodiment, in the reaction promoter, the content (wt%) of the oxy-aluminum composite is in a range of 0.1%-20%, preferably, in a range of 1%-10%, more preferably, in a range of 2%-8%, more preferably, in a range of 4%-7%, more preferably, in a range of 5.5%-6.5%, based on the total weight of the first reaction promoter.

In another preferred embodiment, in the protein synthesis system, the concentration (v/v) of the first reaction promoter is in a range of 20%-70%, preferably, in a range of 30%-60%, more preferably, in a range of 45%-55%, based on the total volume of the protein synthesis system.

In another preferred embodiment, in the protein synthesis system, the content (wt%) of the first reaction promoter is in a range of 20%-70%, preferably, in a range of 30%-60%, more preferably, in a range of 45%-55%, based on the total weight of the protein synthesis system.

According to the second aspect, the present invention provides an *in vitro* cell-free protein synthesis system, comprising:
(a) cell extract;
(b) a first reaction promoter, wherein the first reaction promoter is selected from the group consisting of aluminum, aluminum salt, oxy-aluminum composite, and combinations thereof.

In another preferred embodiment, the protein synthesis system also includes:
(b') a second reaction promoter, wherein the second reaction promoter is selected from the group consisting of monosaccharide, disaccharide, oligosaccharide, soluble starch, and combinations thereof;
(c) polyethylene glycol;
(d) optional exogenous sucrose; and
(e) an optional solvent, wherein the solvent is water or an aqueous solvent.

In another preferred embodiment, the protein synthesis system includes yeast-based *in vitro* protein synthesis system (e.g., *Kluyveromyces-based in vitro* protein synthesis system, preferably *Kluyveromyces lactis* based *in vitro* protein synthesis system).

In another preferred embodiment, the oxy-aluminum composite includes oxy-aluminum nanoparticles.

In another preferred embodiment, the oxy-aluminum composite includes Al₂O₃.

In another preferred embodiment, the oxy-aluminum composite is in a form of particle form or nanoform.

In another preferred embodiment, the particle size of the oxy-aluminum composite is in a range of 0.5-20 mm, preferably in a range of 0.8-10 mm, more preferably in a range of 1-5 mm.

In another preferred embodiment, the average weight of the oxy-aluminum composite is in a range of 1-80 mg, preferably, in a range of 3-50 mg, more preferably, in a range of 4-30 mg.

In another preferred embodiment, in the first reaction promotor, the concentration (v/v) of the oxy-aluminum composite is in a range of 0.5%-20%, preferably, in a range of 0.8%-10%, more preferably, in a range of 1%-10%, more preferably, in a range of 1%-5%, more preferably, in a range of 2%-3%, based on the total volume of the first reaction promoter.

In another preferred embodiment, in the reaction promoter, the content (wt%) of the oxy-aluminum composite is in a range of 0.1%-20%, preferably, in a range of 1%-10%, more preferably, in a range of 2%-8%, more preferably, in a range of 4%-7%, more preferably, in a range of 5.5%-6.5%, based on the total weight of the first reaction promoter.

In another preferred embodiment, in the protein synthesis system, the concentration (v/v) of the first reaction promoter is in a range of 20%-70%, preferably, in a range of 30%-60%, more preferably, in a range of 45%-55%, based on the total volume of the protein synthesis system.

In another preferred embodiment, in the protein synthesis system, the content (wt%) of the first reaction promoter is in a range of 20%-70%, preferably, in a range of 30%-60%, more preferably, in a range of 45%-55%, based on the total weight of the protein synthesis system.

In another preferred embodiment, the cell source of the cell extract is one or more types of cells selected from the group consisting of prokaryotic cells and eukaryotic cells.

In another preferred embodiment, the cell source of the cell extract is one or more types of cells selected from the group consisting of *E. coli,* bacteria, mammalian cells (e.g., HF9 cells, Hela cells, CHO cells, HEK293 cells), plant cells, yeast cells, and combinations thereof.

In another preferred embodiment, the yeast cells are selected from the group consisting of *Saccharomyces cerevisiae* cells, *Pichia pastoris* cells, *Kluyveromyces* cells, and combinations thereof; preferably, the yeast cells include *Kluyveromyces* cells, more preferably, the yeast cells are *Kluyveromyces lactis* cells.

In another preferred embodiment, the protein synthesis system further comprises one or more components selected from the group consisting of:
(f1) substrate for synthesizing RNA;
(f2) substrate for synthesizing protein;
(f3) magnesium ion;
(f4) potassium ion;
(f5) buffer;
(f6) RNA polymerase; and
(f7) energy regeneration system.

In another preferred embodiment, the protein synthesis system further comprises one or more components selected from the group consisting of:
(f8) heme; and
(f9) spermidine.

In another preferred embodiment, the cell extract includes yeast cell extract.

In another preferred embodiment, the yeast cell extract is an aqueous extract of yeast cells.

In another preferred embodiment, the yeast cell extract does not contain yeast endogenous long-chain nucleic acid molecules.

In another preferred embodiment, the yeast cell extract is prepared by using a method comprising the following steps:
(i) providing yeast cells;
(ii) washing the yeast cells to obtain washed yeast cells;
(iii) treating the washed yeast cells with a cell lysis treatment to obtain a crude yeast extract; and
(iv) treating the crude yeast extract via solid-liquid separation to obtain the liquid phase, i.e., the yeast cell extract.

In another preferred embodiment, the solid-liquid separation includes centrifugation.

In another preferred embodiment, the centrifugation is carried out in a liquid state.

In another preferred embodiment, the centrifugation condition is in a range of 5,000 g - 100,000 g, more preferably in a range of 8,000 g - 30,000 g.

In another preferred embodiment, the centrifugation time is in a range from 0.5 minute to 2 hours, and more preferably, from 20 minutes to 50 minutes.

In another preferred embodiment, the centrifugation is carried out at 1-10°C, and more preferably, at 2-6 °C.

In another preferred embodiment, the washing treatment is carried out using a washing solution of pH 7-8 (preferably pH 7.4).

In another preferred embodiment, the washing solution is selected from the group consisting of potassium 4-hydroxyethylpiperazine ethanesulfonate, potassium acetate, magnesium acetate, and combinations thereof.

In another preferred embodiment, methods for cell lysis treatment include high-pressure lysis and freeze-thaw (e.g., treatment at liquid-nitrogen low temperature) lysis.

In another preferred embodiment, the substrate for synthesizing RNA includes: nucleoside monophosphates, nucleoside triphosphates, and a combination thereof.

In another preferred embodiment, the substrate for synthesizing protein includes: 1 to 20 kinds of natural amino acids, and unnatural amino acids.

In another preferred embodiment, the magnesium ion comes from a magnesium ion source, and the magnesium ion source is magnesium acetate, magnesium glutamate, or a combination thereof.

In another preferred embodiment, the potassium ion comes from a potassium ion source, and the potassium ion source is potassium acetate, potassium glutamate, or a combination thereof.

In another preferred embodiment, the energy regeneration system is selected from the group consisting of a phosphocreatine/phosphocreatinase system, glycolysis pathway and its intermediate product energy systems, and combinations thereof.

In another preferred embodiment, the protein synthesis system further comprises (h1) synthetic tRNA.

In another preferred embodiment, the buffer is selected from the group consisting of 4-hydroxyethylpiperazine ethanesulfonic acid, tris(hydroxymethyl) aminomethane, and a combination thereof.

In another preferred embodiment, the protein synthesis system further comprises (i1) an exogenous DNA molecule for guiding protein synthesis.

In another preferred embodiment, the DNA molecule is linear.

In another preferred embodiment, the DNA molecule is circular.

In another preferred embodiment, the DNA molecule contains a sequence encoding an exogenous protein.

In another preferred embodiment, the sequence encoding the exogenous protein includes a genomic sequence and a cDNA sequence.

In another preferred embodiment, the sequence encoding the exogenous protein further comprises a promoter sequence, a 5' untranslated sequence, and/or a 3' untranslated sequence.

In another preferred embodiment, the protein synthesis system comprises components selected from the group consisting of: 4-hydroxyethylpiperazine ethanesulfonic acid, potassium acetate, magnesium acetate, nucleoside triphosphates, amino acids, phosphocreatine, dithiothreitol (DTT), creatine phosphokinase, RNA polymerase, and combinations thereof.

In another preferred embodiment, the polyethylene glycol is selected from the group consisting of: PEG3000, PEG8000, PEG6000, PEG3350, and combinations thereof.

In another preferred embodiment, the polyethylene glycol includes polyethylene glycol with a molecular weight of 200-10,000 Da, preferably, polyethylene glycol with a molecular weight of 3,000-10,000 Da.

In another preferred embodiment, in the protein synthesis system, the concentration (v/v) of the component (a) is in a range from 20% to 70%, preferably from 30% to 60%, more preferably from 40% to 50%, based on the total volume of the protein synthesis system.

In another preferred embodiment, in the protein synthesis system, the content (wt%) of component (c) is in a range from 10% to 95%, preferably from 20% to 80%, more preferably from 40% to 60%, based on the total weight of the protein synthesis system.

In another preferred embodiment, in the protein synthesis system, the concentration (w/v, for example, g/mL) of the component (d) is in a range from 0.1% to 8%, preferably from 0.5% to 4%, and more preferably from 1% to 2%.

In another preferred embodiment, in the protein synthesis system, the concentration of the component (e) is in a range from 0.2% to 4%, preferably from 0.5% to 4%, and more preferably from 0.5% to 1%, based on the total volume of the protein synthesis system.

In another preferred embodiment, the nucleoside triphosphates are selected from the group consisting of adenosine triphosphate, guanosine triphosphate, cytidine triphosphate, uridine triphosphate, and combinations thereof.

In another preferred embodiment, in the protein synthesis system, the concentration of the component (f1) is in a range from 0.1 mM to 5 mM, preferably from 0.5 mM to 3 mM, and more preferably from 1 mM to 1.5 mM.

In another preferred embodiment, the amino acids are selected from the group consisting of glycine, alanine, valine, leucine, isoleucine, phenylalanine, proline, tryptophan, serine, tyrosine, cysteine, methionine, asparagine, glutamine, threonine, aspartic acid, glutamic acid, lysine, arginine, histidine, and combinations thereof.

In another preferred embodiment, the amino acids include amino acids of D-type and/or amino acids of L-type.

In another preferred embodiment, in the protein synthesis system, the concentration of the component (f2) is in a range from 0.01 mM to 0.48 mM, preferably from 0.04 mM to 0.24 mM, more preferably from 0.04 mM to 0.2 mM, and most preferably 0.08 mM.

In another preferred embodiment, in the protein synthesis system, the concentration of the component (f3) is in a range from 1 mM to 10 mM, preferably from 1 mM to 5 mM, and more preferably from 2 mM to 4 mM.

In another preferred embodiment, in the protein synthesis system, the concentration of the component (f4) is in a range from 30 mM to 210 mM, preferably from 30 mM to 150 mM, and more preferably from 30 mM to 60 mM.

In another preferred embodiment, in the protein synthesis system, the concentration of the component (f6) is in a range from 0.01 mg/mL to 0.3 mg/mL, preferably from 0.02 mg/mL to 0.1 mg/mL, and more preferably from 0.027 mg/mL to 0.054 mg/mL.

In another preferred embodiment, in the protein synthesis system, the concentration of 4-hydroxyethylpiperazine ethanesulfonic acid is in a range from 5 mM to 50 mM, preferably from 10 mM to 50 mM, more preferably from 15 mM to 30 mM, and more preferably from 20 mM to 25 mM.

In another preferred embodiment, in the protein synthesis system, the concentration of potassium acetate is in a range from 20 mM to 210 mM, preferably from 30 mM to 210 mM, more preferably from 30 mM to 150 mM, and more preferable from 30 mM to 60 mM.

In another preferred embodiment, in the protein synthesis system, the concentration of magnesium acetate is in a range from 1 mM to 10 mM, preferably from 1 mM to 5 mM, and more preferably from 2 mM to 4 mM.

In another preferred embodiment, in the protein synthesis system, the concentration of phosphocreatine is in a range from 10 mM to 50 mM, preferably from 20 mM to 30 mM, and is more preferably 25 mM.

In another preferred embodiment, in the protein synthesis system, the concentration of heme is in a range from 0.01 mM to 0.1 mM, preferably from 0.02 mM to 0.08 mM, more preferably from 0.03 mM to 0.05 mM, and is most preferably 0.04 mM.

In another preferred embodiment, in the protein synthesis system, the concentration of spermidine is in a range from 0.05 mM to 1 mM, preferably from 0.1 mM to 0.8 mM, more preferably from 0.2 mM to 0.5 mM, more preferably from 0.3 mM to 0.4 mM, and is most preferably 0.04 mM.

In another preferred embodiment, in the protein synthesis system, the concentration of dithiothreitol (DTT) is in a range from 0.2 mM to 15 mM, preferably from 0.2 mM to 7 mM, and more preferably from 1 mM to 2 mM.

In another preferred embodiment, in the protein synthesis system, the concentration of creatine phosphokinase is in a range from 0.1 mg/mL to 1 mg/mL, preferably from 0.2 mg/mL to 0.5 mg/mL, and is more preferably 0.27 mg/mL.

In another preferred embodiment, in the protein synthesis system, the concentration of T7 RNA polymerase is in a range from 0.01 mg/mL to 0.3 mg/mL, preferably from 0.02 mg/mL to 0.1 mg/mL, and more preferably from 0.027 mg/mL to 0.054 mg/mL.

In another preferred embodiment, the protein synthesis system has the following properties:
in the synthesis system, the total amount of synthesized proteins reaches 3 µg of protein per milliliter (mL) of the synthesis system.

In another preferred embodiment, the protein synthesis system comprises the following components:

| | General range | Preferred range |
|---|---|---|
| 4-hydroxyethylpiperazine ethanesulfonic acid | 5-40 mM | 10-30 mM (Preferably, 20-30 mM) |
| magnesium acetate | 1-10 mM | 2-5 mM |
| potassium acetate | 20-150 mM | 30-75 mM |
| DTT | 0.5-5 mM | 1-2 mM |
| phosphocreatine | 15-50 mM | 20-30 mM |
| creatine phosphokinase | 0.1-0.5 mg/mL | 0.2-0.3 mg/mL |
| 4 kinds of ribonucleotides | Respectively 0.5-5 mM | Respectively 1.0-2.0 mM |
| DNA template | 2-50 ng/µL | 5-25 ng/µL |
| RNA polymerase | 0.01-0.3 mg/mL | 0.02-0.10 mg/mL |
| PEG | 0.5-5% (w/v) | 1-3% (w/v) |

In another preferred embodiment, the protein synthesis system also comprises the following components:

| | General range | Preferred range |
|---|---|---|
| spermidine | 0.2-0.4 mM | 0.3-0.4 mM |
| heme | 0.01-0.04 mM | 0.03-0.04 mM |

In another preferred embodiment, the PEG is selected from the group consisting of PEG3350, PEG3000, and/or PEG8000.

In another preferred embodiment, the RNA polymerase is T7 RNA polymerase.

According to the third aspect, the present invention provides a method for producing the protein synthesis system according to the first aspect of the present invention or the second aspect of the present invention, comprising the steps of:
mixing component (i) and component (ii) to obtain the protein synthesis system according to the first aspect or the second aspect of the present invention, wherein the component (i) is selected from the group consisting of cells, cell extract and a combination thereof; the component (ii) is a first reaction promoter, and the first reaction promoter is selected from the group consisting of aluminum, aluminum salt, oxy-aluminum composite, and combinations thereof.

In another preferred embodiment, the component (i) is cell extract.

In another preferred embodiment, in the protein synthesis system, the ratio (mass ratio) of the component (i) to the component (ii) is 0.1-10: 0.1-10, preferably, 0.5-8:0.5-8, more preferably, 0.8-5:0.8-5, more preferably, 0.9-2: 0.9-2.

In another preferred embodiment, in the protein synthesis system, the concentration (v/v) of the component (i) is in a range of 10-80%, preferably in a range of 20-60%, based on the total volume of the protein synthesis system.

In another preferred embodiment, in the protein synthesis system, the content (wt%) of the component (i) is in a range of 10-80%, preferably, in a range of 20-60%, based on the total weight of the protein synthesis system .

In another preferred embodiment, in the protein synthesis system, the concentration (v/v) of the component (ii) is in a range of 10-80%, preferably, in a range of 20-60%, based on the total volume of the protein synthesis system.

In another preferred embodiment, in the protein synthesis system, the content (wt%) of the component (ii) is in a range of 10-80%, preferably, in a range of 20-60%, based on the total weight of the protein synthesis system .

According to the fourth aspect, the present invention provides a method for *in vitro* protein synthesis, wherein, the method comprises steps of:
(i) providing the protein synthesis system according to the first aspect or the second aspect of the present invention, and adding exogenous DNA molecules for guiding protein synthesis; and
(ii) incubating the protein synthesis system provided in the step (i) for a period of time T1 under suitable conditions to synthesize the protein encoded by the exogenous DNA.

In another preferred embodiment, the method further comprises:
(iii) optionally, isolating or detecting the protein encoded by the exogenous DNA from the protein synthesis system.

In another preferred embodiment, the exogenous DNA is derived from a prokaryote or a eukaryote.

In another preferred embodiment, the exogenous DNA is derived from animal, plant or pathogen.

In another preferred embodiment, the exogenous DNA is derived from mammal, preferably from primate or rodent, including human, mouse and rat.

In another preferred embodiment, the coding sequence of the exogenous protein encodes an exogenous protein selected from the group consisting of: luciferin, or luciferase (e.g., firefly luciferase), green fluorescent protein, yellow fluorescent protein, aminoacyl tRNA synthetase, glyceraldehyde-3-phosphate dehydrogenase, catalase, actin, variable regions of antibodies, luciferase mutants, α-amylase, enterocin A, Hepatitis C virus E2 glycoprotein, insulin precursors, interferon αA, interleukin-1β, lysozyme, serum albumins, single-chain variable fragment (scFv) of antibodies, transthyretin, tyrosinase, xylanase, and any combination thereof.

In another preferred embodiment, the exogenous protein is selected from the group consisting of: luciferin, or luciferase (e.g., firefly luciferase), green fluorescent protein, yellow fluorescent protein, aminoacyl tRNA synthetase, glyceraldehyde-3-phosphate dehydrogenase, catalase, actin, variable regions of antibodies, luciferase mutants, α-amylase, enterocin A, Hepatitis C virus E2 glycoprotein, insulin precursors, interferon αA, interleukin-1β, lysozyme, serum albumins, single-chain variable fragment (scFv) of antibodies, transthyretin, tyrosinase, xylanase, and any combination thereof.

In another preferred embodiment, the exogenous DNA encodes an exogenous protein which is selected from the group consisting of: luciferin, or luciferase (e.g., firefly luciferase), green fluorescent protein, yellow fluorescent protein, aminoacyl tRNA synthetase, glyceraldehyde-3-phosphate dehydrogenase, catalase, actin, variable regions of antibodies, luciferase mutants, α-amylase, enterocin A, Hepatitis C virus E2 glycoprotein, insulin precursors, interferon αA, interleukin-1β, lysozyme, serum albumins, single-chain variable fragment (scFv) of antibodies, transthyretin, tyrosinase, xylanase, and any combination thereof.

In another preferred embodiment, the protein encoded by the exogenous DNA is selected from the group consisting of: luciferin, or luciferase (e.g., firefly luciferase), green fluorescent protein, yellow fluorescent protein, aminoacyl tRNA synthetase, glyceraldehyde-3-phosphate dehydrogenase, catalase, actin, variable regions of antibodies, luciferase mutants, α-amylase, enterocin A, Hepatitis C virus E2 glycoprotein, insulin precursors, interferon αA, interleukin-1β, lysozyme, serum albumins, single-chain variable fragment (scFv) of antibodies, transthyretin, tyrosinase, xylanase, and any combination thereof.

In another preferred embodiment, in the step (ii), the reaction temperature is in a range from 20 °C to 37 °C, preferably from 20 °C to 25 °C.

In another preferred embodiment, in the step (ii), the reaction time is in a range from 1 to 6 hours, preferably from 2 to 4 hours.

According to the fifth aspect, the present invention provides a kit, comprising:
(k1) a first container, and component (i) contained in the first container, wherein the component (i) is selected from the group consisting of cells, cell extract, and a combination thereof;
(k2) a second container, and component (ii) contained in the second container, wherein the component (ii) is a first reaction promoter, and the first reaction promoter is selected from the group consisting of aluminum, aluminum salt, oxy-aluminum composite, and combinations thereof; and
(kt) a label or an instruction manual.

In another preferred embodiment, the component (i) is cell extract.

In another preferred embodiment, the first container and the second container are the same container or different containers.

In another preferred embodiment, the kit further includes one or more containers randomly selected from the group consisting of:
(k3) a third container, and polyethylene glycol contained in the third container;
(k4) an optional fourth container, and sucrose contained in the fourth container;
(k5) a fifth container, and substrate for synthesizing RNA which is contained in the fifth container;
(k6) a sixth container, and substrate for synthesizing protein which is contained in the sixth container;
(k7) a seventh container, and magnesium ion contained in the seventh container;
(k8) an eighth container, and potassium ion contained in the eighth container; and
(k9) a ninth container, and buffer contained in the ninth container.

It should be understood that, within the scope of the present invention, the above-mentioned technical features and the technical features specifically described hereinafter (e.g., embodiments or examples) can be combined with each other, whereby forming new or preferred technical solutions. For the simplicity, details will not be repeated herein.

### BRIEF DESCRIPTION OF THE DRAWINGS

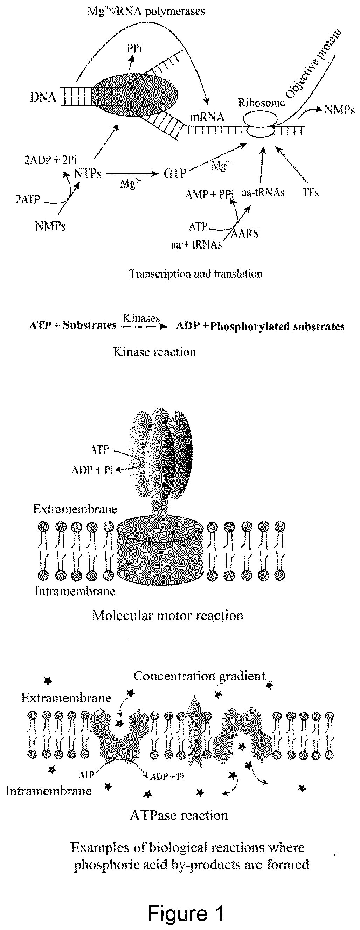

Figure 1 shows the release of reaction by-products represented by phosphate ion in biological reactions. For example, in several steps of transcription and translation, magnesium ion is involved, and the main objective product is produced through the reaction using substrates, energy and so on in a solid state, a liquid state or another phase state; meanwhile, in several stages of transcription and translation, several reaction by-products, such as free phosphate ion and pyrophosphate ion, are generated; for example, in kinase (protein kinase, lipid kinase, glycokinase, etc.) reaction; in ATPase reaction (transmembrane transport of sodium ion, potassium ion, calcium ion, etc.), molecular motor reaction and so on, by-products are often counteractive to biological reaction systems and inhibit the reaction. At present, known methods for removing reaction by-products mainly include dialysis method, circulation method, solution replacement method, etc; those known methods all need additional apparatus, have complex systems, are not easy to be operated and are difficult to be completed in small reaction systems. Therefore, the removal of reaction by-products, especially *in situ* removal thereof is the key to improve the reaction efficiency and to reduce the reaction complexity.

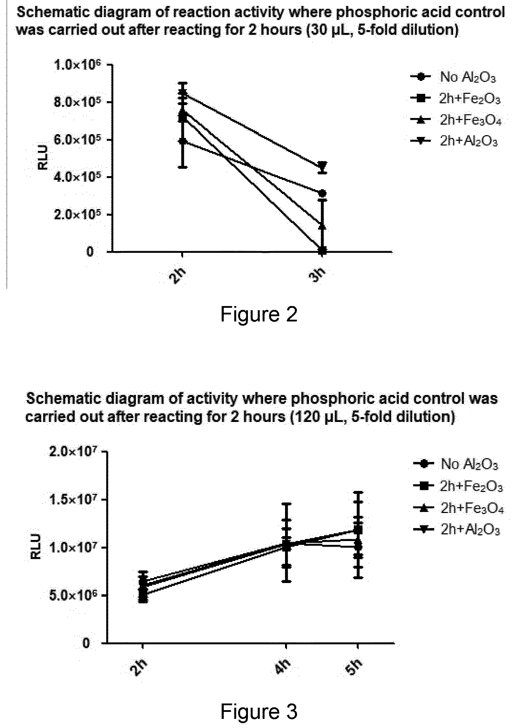

Figure 2 shows a schematic diagram of the effect of adding the first reaction promoter (phosphoric acid control agent) into a 30 µL *in vitro* protein synthesis system at 2 hour of reaction. The solid circle line is a cell-free reaction system without the addition of the first reaction promoter; the square is the cell-free reaction system with Fe₂O₃ being added at 2 hour of reaction; the regular triangle is the cell-free reaction system with equal mass of Fe₃O₄ being added at 2 hour of reaction; and the inverted triangle is the cell-free reaction system with equal mass of aluminum oxide particles being added at 2 hour of reaction. The reactions were carried out at 20-25 °C for 3 hours. All errors are standard deviations obtained from three repetitions.
Figure 3 shows a schematic diagram of the effect of adding the first reaction promoter into a 120 µL cell-free reaction system at 2 hour of reaction. The solid circle line is the cell-free reaction system without the addition of the first reaction promoter; the square is the cell-free reaction system with Fe₂O₃ being added at 2 hour of reaction; the regular triangle is the cell-free reaction system with equal mass of Fe₃O₄ being added at 2 hour of reaction; and the inverted triangle is the cell-free reaction system with equal mass of aluminum oxide particles being added at 2 hour of reaction. The reactions were carried out at 20-25 °C for 4 hours and 5 hours, respectively. All errors are standard deviations obtained from three repetitions.

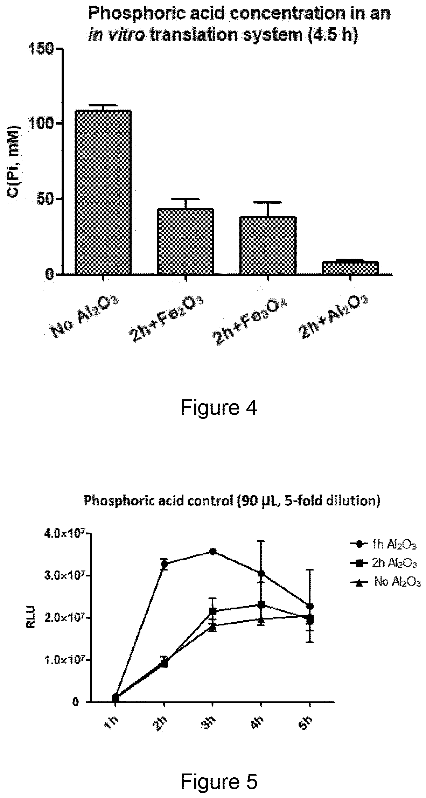

Figure 4 shows a schematic diagram in which the first reaction promoter was added into a 120 µL cell-free reaction system at 2 hour of reaction and the phosphoric acid concentration in the system was measured at 4.5 hour of reaction. The first column is the cell-free reaction system without adding the first reaction promoter; the second column is the cell-free reaction system with Fe₂O₃ being added at 2 hour of reaction; the third column is the cell-free reaction system with equal mass of Fe₃O₄ being added at 2 hour of reaction; and the fourth column is the cell-free reaction system with equal mass of aluminum oxide particles being added at 2 hour of reaction. The reactions were carried out at 20-25 °C for 4.5 hours.
Figure 5 shows a schematic diagram of the effect of adding 30 mg of aluminum oxide particles into a 90 µL cell-free reaction system at different time periods (at 1 hour and at 2 hour respectively) of reaction and of the effect of no addition of 30 mg aluminum oxide particles. The reactions were carried out at 20-25 °C. All errors are standard deviations obtained from three repetitions.

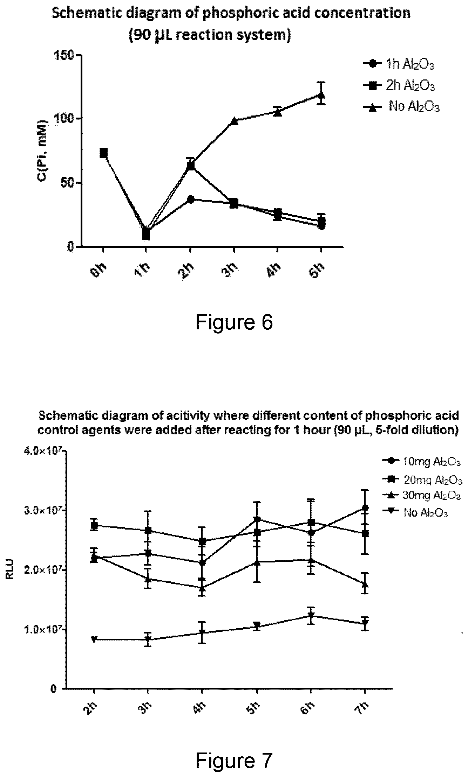

Figure 6 shows a schematic diagram of phosphoric acid concentration in a 90 µL cell-free reaction system in which 30 mg aluminum oxide particles was added at different time periods (at 1 hour and at 2 hour respectively) of reaction, and also in which 30 mg aluminum oxide particles were not added. The reactions were carried out at 20-25 °C. All errors are standard deviations obtained from three repetitions.
Figure 7 shows the schematic diagram of the effect of adding different mass of aluminum oxide particles into a 90 µL cell-free reaction system at 1 hour of reaction. The reaction conditions were 20-25 °C. All errors are standard deviations obtained from three repetitions.

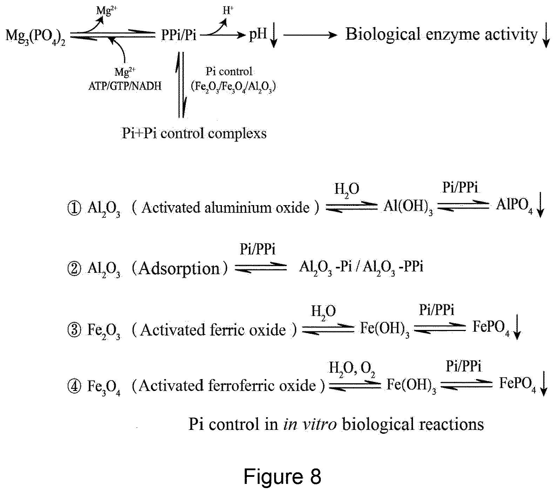

Figure 8 is a schematic diagram of enhancing the efficacy of biological reactions by using the phosphoric acid control system (a system for controlling phosphoric acid). In short, the phosphoric acid control in the system and the biochemical reaction in the system are a pair of reactions competing with each other. In the system, excessive phosphate ion or pyrophosphate ion would cause pH imbalance in the system, affect the activity of biological enzymes in the system which participate in biochemical reactions, and reduce the yield of objective proteins; at the same time, phosphate ion is liable to combine with magnesium ion requisite for the reaction system to form magnesium sulfate which is hardly soluble in water, leading to the reduction of reaction activity of many biochemical reaction steps because of the lack of magnesium ion. The addition of phosphoric acid control agent adjusts the balance of the biochemical reactions as described above through physical manner (adsorption) or chemical manner (forming precipitate which can be separated from the reaction system), thereby creating a relatively ideal reaction environment for the protein synthesis system.

### DETAILED DESCRIPTION

After extensive and in-depth research, the inventor unexpectedly found for the first time that the *in vitro* protein synthesis system formed by mixing (a) cells, cell extract, or a combination thereof; and (b) a first reaction promoter (such as aluminum, aluminum salt, oxy-aluminum composite, and any combination thereof) at a specific ratio could significantly improve the synthesis efficiency of exogenous proteins, and RLU value of the exogenous proteins can reach as high as 3×10⁹-4×10⁹. Under the action of the first reaction promoter, the RLU value of the exogenous proteins (e.g., luciferases) synthesized by the yeast-based cell-free expression system with the addition of the first reaction promoter after reacting for 1 hour compared with that by the yeast-based cell-free expression system without the addition of the first reaction promoter, was significantly increased by 2-3 times; the reaction efficiency was raised and the yield of the objective proteins was improved. Based on the above, the inventors have completed the present invention.

In addition, the experimental results of the present invention show that a cell-free *in vitro* protein synthesis system with the first reaction promoter being added at a specific time period (such as when the reaction lasts for about 1 hour) can significantly promote the *in vitro* protein synthesis in the protein synthesis system (e.g., yeast-based *in vitro* protein synthesis system). And the first reaction promoter of the present invention can be added at the beginning of the reaction, at 1 hour after the beginning of the reaction and at 2 hour after the beginning of the reaction of the protein synthesis system (e.g., yeast-based *in vitro* protein synthesis system). Wherein, adding the first reaction promoter at 1 hour after the beginning of the reaction of the protein synthesis system (e.g., yeast-based *in vitro* protein synthesis system) promoted protein synthesis better.

### REACTION PROMOTER

In the present invention, the reaction promoter is not particularly limited, and reaction promoters, as long as they can significantly improve the protein synthesis efficiency of the *in vitro* protein synthesis system of the present invention, are within the protection scope of the present invention.

In a preferred embodiment, the reaction promoter of the present invention includes a first reaction promoter and a second reaction promoter.

In a preferred embodiment, the first reaction promoter is selected from the group consisting of aluminum, aluminum salt, oxy-aluminum composite, and combinations thereof.

In a preferred embodiment, the second reaction promoter is selected from the group consisting of monosaccharide, disaccharide, oligosaccharide, soluble starch, and combinations thereof.

In a preferred embodiment, the second reaction promoter is one or more kinds of substances selected from the following group consisting of:
a) sugars/polyols: sucrose, trehalose, mannitol, lactose, glucose, maltose, etc.;
b) polymers: polyethylene glycol (PEG), glucan, albumin, etc.; and
c) anhydrous solvent: glycerin, DMSO, etc.

In a preferred embodiment, the second reaction promoter is selected from the group consisting of sucrose, trehalose, mannitol, lactose, glucose, maltose, galactose, and combinations thereof.

In a preferred embodiment, the second reaction promoter is selected from the group consisting of sucrose, trehalose, lactose, and combinations thereof.

### BIOLOGICAL REACTIONS

Biological reactions, i.e., biochemical reactions, refer to chemical reactions that take place in organisms. These reactions are catalyzed by enzymes. Enzymes and reactants should be dissolved in the water of the internal environment before the reaction begins. Water is provided as a carrier and a medium for *in vivo* substances. In the last few decades of the 20^{th} century, biochemistry has made great achievements in explaining life processes, and now almost all fields related to life sciences such as botany, medicine and genetics are engaged in biochemical research. The biochemical reactions that take place in organisms or cells can be automatically regulated by positive and negative feedback in a complex network system. The biochemical reactions that take place inside cells all need to be catalyzed by enzymes. The enzymes have high catalytic efficiency, mild reaction conditions, directionality and high specificity for substrates.

### CELL EXTRACT

In a preferred embodiment, the cell source of the cell extract is one or more types of cells selected from the group consisting of prokaryotic cells and eukaryotic cells.

In a preferred embodiment, the cell source of the cell extract is one or more types of cells selected from the group consisting of *E. coli,* bacteria, mammalian cells (e.g., HF9 cells, Hela cells, CHO cells, HEK293 cells), plant cells, yeast cells, insect cells, and combinations thereof.

In a preferred embodiment, the yeast cells are selected from the group consisting of *Saccharomyces cerevisiae* cells, *Pichia pastoris* cells, *Kluyveromyces* cells, and combinations thereof; preferably, the yeast cells include *Kluyveromyces* cells, more preferably, the yeast cells are *Kluyveromyces lactis* cells.

In the present invention, the cell extract includes yeast cell extract.

In the present invention, the content and purity of the cell extract are not particularly limited.

In a preferred embodiment, in the protein synthesis system, the content (wt%) of the cell extract (e.g., yeast cell extract) is in a range of 10%-95%, preferably, in a range of 20%-80%, more preferably, in a range of 40%-60%, based on the total weight of the protein synthesis system.

### IN VITRO EXPRESSION SYSTEM

Yeast has advantages of simple culture, efficient protein folding and post-translational modification. Among yeasts, *Saccharomyces cerevisiae* and *Pichia pastoris* are model organisms for expressing complex eukaryotic proteins and membrane proteins. In addition, yeast can also be used as a raw material for the preparation of an *in vitro* translation system.

*Kluyveromyces* is an ascosporogenous yeast. Among *Kluyveromyces, Kluyveromyces marxianus* and *Kluyveromyces lactis* are yeasts widely used in industry. Compared with other yeasts, *Kluyveromyces lactis* has many advantages, such as super secretion ability, better large-scale fermentation characteristics, conforming to food safety level, having the ability of post-translational modification of proteins, etc.

In the present invention, the yeast-based *in vitro* expression system is not particularly limited. A preferred yeast-based *in vitro* expression system is a *Kluyveromyces-*based expression system (more preferably, a *Kluyveromyces lactis* based expression system).

### CONTROLLING THE CONTENT OF FREE PHOSPHATE ION IN THE PROTEIN SYNTHESIS SYSTEM

In the present invention, "controlling the content of free phosphate ion in the protein synthesis system" refers to controlling the free phosphate ion, one of the reaction by-products in the protein synthesis system, adjusting the influence on the protein synthesis system, and thereby improving the protein synthesis efficiency in the protein synthesis system.

In the present invention, the reaction promoter of the present invention can be added into the protein synthesis system to achieve the above-mentioned object.

In a preferred embodiment, the free phosphorus content control stages include a pre-reaction stage before phosphoric acid control, a phosphoric acid content control stage and a post-stage under phosphoric acid control.

In a preferred embodiment, the phosphoric acid content control stage has no particular time limit, and the amount of free phosphorus control agent to be added is determined according to the time of the reaction.

In a preferred embodiment, the time of the pre-reaction stage before phosphoric acid control is 0±2 hours, preferably, 1 hour; the time of the phosphoric acid content control stage is 0±5 hours after the pre-reaction stage before phosphoric acid control, preferably, 2-5 hours; the duration of the post-stage under phosphoric acid control is determined according to respective objective proteins and biochemical reaction characteristics. In summary, the duration of the entire phosphoric acid control reaction system is 1-7 hours, preferably 2-4 hours.

### IN VITRO CELL-FREE PROTEIN SYNTHESIS SYSTEM

In a preferred embodiment, the *in vitro* cell-free protein synthesis system of the present invention includes a yeast-based *in vitro* protein synthesis system.

Yeast has advantages of simple culture, efficient protein folding and post-translational modification. Among yeasts, *Saccharomyces cerevisiae* and *Pichia pastoris* are model organisms for expressing complex eukaryotic proteins and membrane proteins. In addition, yeast can also be used as a raw material for the preparation of an *in vitro* translation system.

*Kluyveromyces* is an ascosporogenous yeast. Among *Kluyveromyces, Kluyveromyces marxianus* and *Kluyveromyces lactis* are yeasts widely used in industry. Compared with other yeasts, *Kluyveromyces lactis* has many advantages, such as super secretion ability, better large-scale fermentation characteristics, conforming to food safety level, having the ability of post-translational modification of proteins, etc.

In the present invention, the yeast-based *in vitro* protein synthesis system is not particularly limited. A preferred yeast-based *in vitro* protein synthesis system is a *Kluyveromyces-*based expression system (more preferably, a *Kluyveromyces lactis* based expression system).

In the present invention, *Kluyveromyces* (e.g., *Kluyveromyces lactis*) is not particularly limited, and includes any strain of *Kluyveromyces* (e.g., *Kluyveromyces lactis*) that can improve the efficiency of synthesizing proteins.

In the present invention, the yeast-based *in vitro* protein synthesis system comprises:
(a) cell extract (e.g., yeast cell extract);
(b) a first reaction promoter, wherein the first reaction promoter is selected from the group consisting of aluminum, aluminum salt, oxy-aluminum composite, and combinations thereof.

In a preferred embodiment, the yeast-based *in vitro* protein synthesis system further comprises:
(b') a second reaction promoter, wherein the second reaction promoter is selected from the group consisting of monosaccharide, disaccharide, oligosaccharide, soluble starch, and combinations thereof;
(c) polyethylene glycol;
(d) optional exogenous sucrose; and
(e) an optional solvent, wherein the solvent is water or an aqueous solvent.

In a preferred embodiment, the particle size of the oxy-aluminum composite is in a range of 0.5-20 mm, preferably, in a range of 0.8-10 mm, more preferably, in a range of 1-5 mm, more preferably, in a range of 1-3 mm.

In another preferred embodiment, the average weight of the oxy-aluminum composite is in a range of 1-80 mg, preferably, in a range of 3-50 mg, more preferably, in a range of 4-30 mg, more preferably, in a range of 8-15 mg.

In another preferred embodiment, in the reaction promotor, the concentration (v/v) of the oxy-aluminum composite is in a range of 0.5%-20%, preferably, in a range of 0.8%-10%, more preferably, in a range of 1%-10%, more preferably, in a range of 1%-5%, more preferably, in a range of 2%-3%, based on the total volume of the first reaction promoter.

In another preferred embodiment, in the reaction promoter, the content (wt%) of the oxy-aluminum composite is in a range of 0.1%-20%, preferably, in a range of 1%-10%, more preferably, in a range of 2%-8%, more preferably, in a range of 4% -7%, more preferably, in a range of 5.5%-6.5%, based on the total weight of the first reaction promoter.

In another preferred embodiment, in the protein synthesis system, the concentration (v/v) of the first reaction promoter is in a range of 20%-70%, preferably, in a range of 30%-60%, more preferably, in a range of 45%-55%, based on the total volume of the protein synthesis system.

In another preferred embodiment, in the protein synthesis system, the content (wt%) of the first reaction promoter is in a range of 20%-70%, preferably, in a range of 30%-60%, more preferably, in a range of 45%-55%, based on the total weight of the protein synthesis system.

In another preferred embodiment, in the protein synthesis system, the ratio (mass ratio) of component (i) to component (ii) is 0.1-10: 0.1-10, preferably, 0.5-8: 0.5-8, more preferably, 0.8-5: 0.8-5, more preferably, 0.9-2: 0.9-2.

In a particularly preferred embodiment, the *in vitro* protein synthesis system provided by the present invention comprises one or more or all components selected from the group consisting of: yeast cell extract, oxy-aluminum composite (e.g., aluminum oxide), polyethylene glycol, sucrose, 4-hydroxyethylpiperazine ethanesulfonic acid, potassium acetate, magnesium acetate, adenosine triphosphate (ATP), guanosine triphosphate (GTP), cytidine triphosphate (CTP), thymidine triphosphate (TTP), amino acid mixture, phosphocreatine, dithiothreitol (DTT), creatine phosphokinase, RNase inhibitor, luciferin, DNA of luciferase, RNA polymerase, spermidine and heme.

In the present invention, the RNA polymerase is not particularly limited. The RNA polymerase can be one or more RNA polymerases, and a typical RNA polymerase is T7 RNA polymerase.

In the present invention, the proportion of the yeast cell extract in the *in vitro* protein synthesis system is not particularly limited. Generally, the content (wt%) of the yeast cell extract is in a range of 10%-95%, preferably, in a range of 20%-80%, more preferably, in a range of 40%-60%, based on the total weight of the protein synthesis system.

In the present invention, the yeast cell extract does not contain intact cells, and a typical yeast cell extract comprises: substances used for translation including ribosome, transfer RNA and aminoacyl tRNA synthetase, and factors required for protein synthesis including initiation factors, elongation factors and termination release factors (release factors mediating termination). Furthermore, the yeast extract also comprises some other proteins derived from the cytoplasm of yeast cells, especially soluble proteins.

In the present invention, the protein content of the yeast cell extract is 20-100 mg/mL, preferably 50-100 mg/mL. The method for measuring the protein content is Coomassie brilliant blue assay.

In the present invention, the method for preparing the yeast cell extract is not particularly limited. A preferred preparation method comprises the following steps:
(i) providing yeast cells;
(ii) washing the yeast cells to obtain washed yeast cells;
(iii) treating the washed yeast cells with a cell lysis treatment to obtain a crude yeast extract; and
(iv) treating the crude eukaryotic extract via solid-liquid separation to obtain the liquid phase, that is the yeast cell extract.

In the present invention, the method of solid-liquid separation is not particularly limited. A preferred method is centrifugation.

In a preferred embodiment, the centrifugation is carried out in a liquid state.

In the present invention, the centrifugation condition is not particularly limited. A preferred centrifugation condition is in a range of 5,000 g - 100,000 g, more preferably in a range of 8,000 g - 30,000 g.

In the present invention, the centrifugation time is not particularly limited. A preferred centrifugation time is in a range from 0.5 minute to 2 hours, preferably, from 20 minutes to 50 minutes.

In the present invention, the centrifugation temperature is not particularly limited. Preferably, the centrifugation is carried out at 1-10 °C, more preferably 2-6 °C.

In the present invention, the washing treatment method is not particularly limited. A preferred washing treatment method is to carry out the treatment using a washing solution of pH 7-8 (preferably, pH 7.4). The washing solution is not particularly limited. A typical washing solution is selected from the group consisting of potassium 4-hydroxyethylpiperazine ethanesulfonate, potassium acetate, magnesium acetate, and combinations thereof.

In the present invention, the method for cell lysis treatment is not particularly limited. A preferred method for cell lysis treatment includes high-pressure lysis and freeze-thaw (e.g., treatment at liquid-nitrogen low temperature) lysis.

The mixture of nucleoside triphosphates in the *in vitro* protein synthesis system comprises adenosine triphosphate, guanosine triphosphate, cytidine triphosphate, and uridine triphosphate. In the present invention, there is no limitation to the concentration of each single nucleotide. Generally, the concentration of each single nucleotide is in a range of 0.5 mM to 5 mM, preferably in a range of 1.0 mM to 2.0 mM.

The amino acid mixture in the *in vitro* protein synthesis system may comprise natural or unnatural amino acids, and may include amino acids of D-type or amino acids of L-type. Representative amino acids include, but are not limited to, 20 types of natural amino acids: glycine, alanine, valine, leucine, isoleucine, phenylalanine, proline, tryptophan, serine, tyrosine, cysteine, methionine, asparagine, glutamine, threonine, aspartic acid, glutamic acid, lysine, arginine, and histidine. The concentration of each type of amino acid is usually in a range from 0.01 mM to 0.5 mM, preferably, from 0.02 mM to 0.2 mM, such as 0.05 mM, 0.06 mM, 0.07 mM, and 0.08 mM.

In a preferred embodiment, the *in vitro* protein synthesis system further comprises polyethylene glycol (PEG) or analogs thereof. The concentration of polyethylene glycol or analogs thereof is not particularly limited. Generally, the concentration (w/v) of polyethylene glycol or analogs thereof is in a range from 0.1% to 8%, preferably from 0.5% to 4%, more preferably from 1% to 2%, based on the total weight of the protein synthesis system. Representative examples of PEG include, but are not limited to, PEG3000, PEG8000, PEG6000 and PEG3350. It should be understood that the system of the present invention may also include polyethylene glycols of other various molecular weights (such as PEG 200, 400, 1500, 2000, 4000, 6000, 8000, 10000, etc.).

In a preferred embodiment, the *in vitro* protein synthesis system further comprises sucrose. The concentration of sucrose is not particularly limited. Generally, the concentration (w/v) of sucrose is in a range of 0.2-4%, preferably in a range of 0.5-4%, more preferably in a range of 0.5-1%, based on the total volume of the protein synthesis system.

In a preferred embodiment, the *in vitro* protein synthesis system further comprises heme. The concentration of heme is not particularly limited. Generally, the concentration of heme is in a range of 0.01-0.1 mM, preferably, in a range of 0.02-0.08 mM, more preferably, in a range of 0.03-0.05 mM, and most preferably, the concentration of heme is 0.04 mM.

In a preferred embodiment, the *in vitro* protein synthesis system further comprises spermidine. The concentration of spermidine is not particularly limited. Generally, the concentration of spermidine is in a range of 0.05-1 mM, preferably, in a range of 0.1-0.8 mM, more preferably, in a range of 0.2-0.5 mM, more preferably, in a range of 0.3-0.4 mM, and most preferably, the concentration of spermidine is 0.4 mM.

In a preferred embodiment, the *in vitro* protein synthesis system further comprises a buffer, and the components of the buffer are not particularly limited. A preferred buffer contains 4-hydroxyethylpiperazineethanesulfonic acid and/or Tris buffer liquid. In the present invention, the buffer may further comprise other buffer components, such as potassium acetate and magnesium acetate, so as to form a reaction solution or reaction buffer with a pH value of 6.5-8.5 (preferably pH 7.0-8.0). In the present invention, the type and content of the buffer are not particularly limited. Generally, the concentration of the buffer is in a range of 1-200 mM or 1-100 mM, preferably, in a range of 5-50 mM.

A particularly preferred *in vitro* protein synthesis system, in addition to the yeast extract and oxy-aluminum composite (e.g., aluminium oxide), further comprises one or more or all components selected from the group consisting of: 22 mM 4-hydroxyethylpiperazine ethanesulfonic acid (pH 7.4), 30-150 mM potassium acetate, 1.0-5.0 mM magnesium acetate, 1.5-4 mM nucleoside triphosphate mixture, 0.08-0.24 mM amino acid mixture, 25 mM phosphocreatine, 1.7 mM dithiothreitol, 0.27 mg/mL creatine phosphokinase, 1%-4% polyethylene glycol, 0.5%-2% sucrose, 8-20 ng/µL DNA of firefly luciferase, 0.027-0.054 mg/mL T7 RNA polymerase, 0.03-0.04 mM heme, and 0.3-0.4 mM spermidine.

### CODING SEQUENCE OF EXOGENOUS PROTEINS (EXOGENOUS DNA)

As used herein, the terms "coding sequence of an exogenous protein" and "exogenous DNA" can be used interchangeably, and both refer to exogenous DNA molecules for guiding protein synthesis. The DNA molecules are generally linear or circular. The DNA molecules contain sequences encoding exogenous proteins. In the present invention, examples of sequences encoding exogenous proteins include, but are not limited to, genomic sequences and cDNA sequences. The sequences encoding exogenous proteins further comprise promoter sequence, 5' untranslated sequence, and/or 3' untranslated sequence.

In the present invention, the selection of the exogenous DNA is not particularly limited. Generally, the exogenous DNA is selected from the group consisting of exogenous DNAs encoding luciferin, or luciferase (e.g., firefly luciferase), green fluorescent protein, yellow fluorescent protein, aminoacyl tRNA synthetase, glyceraldehyde-3-phosphate dehydrogenase, catalase, actin and variable regions of antibodies, DNA of a luciferase mutant, and any combination thereof.

The exogenous DNA may also be selected from the group consisting of exogenous DNAs encoding α-amylase, enterocin A, Hepatitis C virus E2 glycoprotein, insulin precursors, interferon αA, interleukin-1β, lysozyme, serum albumins, single-chain variable fragment (scFv) of antibodies, transthyretin, tyrosinase and xylanase, and any combination thereof.

In a preferred embodiment, the exogenous DNA encodes a protein which is selected from the group consisting of: green fluorescent protein (enhanced GFP, eGFP), yellow fluorescent protein (YFP), *E. coli* β-galactosidase (LacZ), human lysine-tRNA synthetase, human leucine-tRNA synthetase, Arabidopsis thaliana glyceraldehyde-3-phosphate dehydrogenase, murine catalase, and any combination thereof.

### KIT

The present invention provides a kit for *in vitro* protein synthesis, comprising:
(k1) a first container, and component (i) contained in the first container, wherein the component (i) is selected from the group consisting of cells, cell extract, and a combination thereof;
(k2) a second container, and component (ii) contained in the second container, wherein the component (ii) is a first reaction promoter, and the first reaction promoter is selected from the group consisting of aluminum, aluminum salt, oxy-aluminum composite, and combinations thereof; and
(kt) a label or an instruction manual.

In a preferred embodiment, the first container and the second container are the same container or different containers.

The present invention also provides a kit for cell-free *in vitro* protein synthesis, comprising:
(k1) a first container, and component (i) contained in the first container, wherein the component (i) is cell extract;
(k2) a second container, and component (ii) contained in the second container, wherein the component (ii) is a first reaction promoter, and the first reaction promoter is selected from the group consisting of aluminum, aluminum salt, oxy-aluminum composite, and combinations thereof; and
(kt) a label or an instruction manual.

A particularly preferred kit for *in vitro* protein synthesis contains an *in vitro* protein synthesis system; the *in vitro* protein synthesis system comprises one or more or all components selected from the group consisting of: yeast cell extract, oxy-aluminum composite (e.g., aluminum oxide), 4-hydroxyethylpiperazineethanesulfonic acid, potassium acetate, magnesium acetate, adenosine triphosphate (ATP), guanosine triphosphate (GTP), cytidine triphosphate (CTP), thymidine triphosphate (TTP), amino acid mixture, phosphocreatine, dithiothreitol (DTT), creatine phosphokinase, RNase inhibitor, luciferin, DNA of luciferase, T7 RNA polymerase, spermidine and heme.

The main advantages of the present invention include as follows,
(1) Compared with general *in vitro* protein synthesis systems, the *in vitro* protein synthesis system of the present invention comprising the first reaction promoter has an obvious advantage that the protein synthesis capacity can be improved by 2-3 times.
(2) The *in vitro* protein synthesis system of the present invention can more conveniently and more quickly control biochemical reactions, such as enzyme reactions, biosynthesis reactions, biodegradation reactions, cell-free biological reactions, etc.
(3) The present invention found for the first time that adding a reaction promoter into an *in vitro* protein synthesis system can control the concentration of phosphate ion in the protein synthesis system and can significantly improve protein synthesis efficiency.
(4) The present invention used *in situ* removal method for the first time to improve biological reaction efficiency and reduce reaction complexity.

The present invention will be further illustrated below in combination with specific examples (or embodiments). It should be understood that these examples (or embodiments) are provided solely for the purpose of illustration and should not to be regarded as limitations to the scope of the present invention. With respect to the experimental methods without specifically described conditions in the following examples (or embodiments), one person may generally follow conventional conditions, such as conditions described in Sambrook et.al, Molecular Cloning: A Laboratory Manual (New York: Cold Spring Harbor Laboratory Press, 1989), or follow the conditions recommended by the manufacturer. Unless otherwise stated, percentage and portions refer to percentage by weight and portions by weight.

Unless otherwise stated, materials and reagents used in the examples of the present invention are all commercially available products.

### GENERAL METHOD

In the present invention, after the yeast extract is obtained, sucrose and/or mannitol is added into the yeast extract, and the resulting mixture is then lyophilized to obtain a lyophilized yeast extract.

In the present invention, the biological reaction system is exemplified by an *in vitro* protein synthesis system, and the biological reactants are exemplified by yeast cell extracts, but are not limited thereto.

### Example 1 Preparation of Yeast Cell Extract by Liquid Nitrogen Lysis Method

1.1. First-level seed culture: the strain frozen at -80 °C was inoculated into culture medium in a shake flask, and cultured at 30 °C and 200 rpm until reaching the logarithmic growth phase.
1.2. Second-level seed culture: appropriate amount of the first-level seed yeast solution was taken out, inoculated into second-level seed, and then cultured at 30 °C and 200 rpm until reaching the logarithmic growth phase.
1.3. Batch culture stage: the second-level seed yeast solution was inoculated into a fermenter, the temperature was controlled at 30 °C for a 10-12 hour culture, and then the culture came into a feeding culture stage. When the OD600 value became 50-55, the cell culture was collected.
1.4. The cell culture having been cultured well was pre-cooled in ice/water mixture for 10-30 minutes.
1.5. The cell culture already pre-cooled in the step 1.4 was centrifuged at 3,000 g, at 4 °C for 10 minutes in a low-temperature centrifuge to obtain yeast cells.
1.6. The yeast cells obtained in the step 1.5 were resuspended with pre-cooled washing buffer, and the resulting resuspended liquid was centrifuged at 3000 g, at 4 °C for 10 minutes to obtain yeast cells. The compositions of the washing buffer comprised: 20-30 mM potassium 4-hydroxyethylpiperazine ethanesulfonate (pH 7.4), 100-150 mM potassium acetate, and 1-4 mM magnesium acetate;
1.7. Step 1.6 was repeated 2 to 3 times.
1.8. The yeast cells obtained in the step 1.7 were directly subjected to subsequent operations, or were quickly frozen with liquid nitrogen and then stored at -80 °C.
1.9. Using a liquid nitrogen homogenizer to perform a cell lysis treatment: appropriate amount of liquid nitrogen was added into the homogenizer, then the yeast cells obtained by centrifugation or the yeast cells stored at -80 °C in the step 1.8 were added into the homogenizer and disrupted for 3-10 minutes at a speed of 45,000 rpm. The low-temperature powder having been disrupted well was aliquoted into 50 mL centrifuge tubes, weighed and stored at -80 °C for use.
1.10. The disruption powder of yeast cells obtained in the step 1.9 was cooled down to 4 °C from room temperature condition, and each gram of cell disruption powder was dissolved in 0.2-1 mL of lysis buffer pre-cooled at 4 °C to obtain a crude extract of yeast cells. The lysis buffer consisted of 10-40 mM potassium 4-hydroxyethylpiperazine ethanesulfonate (pH 7.4), 50-150 mM potassium acetate, 1-4 mM magnesium acetate, 2-7 mM dithiothreitol, and 0.5-2 mM phenylmethylsulfonyl fluoride.
1.11. The crude extract of yeast cells harvested in the step 1.10 was centrifuged 1-2 times; the centrifugation force was in a range of 12,000-30,000 g, the centrifugation time was 30 minutes, and the centrifugation temperature was 4 °C;
1.12. After centrifugation, the upper clear liquid was yeast cell extract.
1.13. The prepared yeast cell extract was aliquoted, quickly frozen in liquid nitrogen and then stored at -80 °C.

### Example 2 Lyophilization of Cell Extract

2.1 The yeast extract stored at -80 °C was placed under room temperature condition and thawed;
2.2 Each glass bottle or each glass plate was weighed and corresponding weight values were recorded respectively;
2.3 The thawed yeast extract was aliquoted into the glass bottles or the glass plates, each glass bottle containing about 500 microliters of cell extract, or each glass plate containing about 5 milliliters of cell extract.
2.4 The glass bottles and glass plates containing the cell extract in the step 2.3 were weighed again and corresponding weight values were recorded;
2.5 The glass bottles and the glass plates containing the cell extract in the step 2.4 were pre-frozen at -80 °C for 2-4 hours;
2.6 Setting the lyophilization program: pre-freeze stage: -55 °C, 4 hours; the first stage of lyophilization: -30 °C, 4-10 hours; the second stage of lyophilization: -30 °C, 10 hours; the final stage of lyophilization: -20 °C, 10-20 hours.
2.7 The pre-frozen glass bottles and glass plates containing the cell extract in the step 2.5 were placed on the plate of the lyophilizer, and the lyophilization procedure was carried out with a vacuum degree of about 0.1 MPa.
2.8 The lyophilized cell extract was stored at -80 °C, or directly added with water of equal mass relative to the cell extract before lyophilization, and then the lyophilized cell extract and water were mixed well to perform subsequent protein *in vitro* synthesis activity assay.

### Example 3 in vitro Biological Reaction System

3.1 Preparation of stock solution for the *in vitro* biological reaction system: 1 M Tris-HCl (pH 8.0); 5 M potassium acetate; 1 M magnesium acetate; 25 mM of a mixture of four nucleoside triphosphates, including adenosine triphosphate, guanosine triphosphate, cytidine triphosphate and uridine triphosphate; 1 mM of a mixture of 20 types of amino acids, including glycine, alanine, valine, leucine, isoleucine, phenylalanine, proline, tryptophan, serine, tyrosine, cysteine, methionine, asparagine, glutamine, threonine, aspartic acid, glutamic acid, lysine, arginine and histidine, wherein, the concentration of each type of amino acids was 1.0 mM; 1 M glucose; 1 M dithiothreitol; 1 M potassium phosphate; 2.4 mg/mL T7 RNA polymerase; 20%-50% polyethylene glycol (PEG) 3350 or polyethylene glycol (PEG) 8000; 1-4 mM spermidine; and 0.1-0.4 mM heme;
3.2 The *in vitro* protein synthesis reaction system (final concentration): 22 mM of Tris-HCI with pH of 7-9, 30-150 mM potassium acetate, 1.0-5.0 mM magnesium acetate, 1.5-4 mM of a mixture of nucleoside triphosphates (adenosine triphosphate, guanosine triphosphate, cytidine triphosphate and uridine triphosphate), 0.08-0.24 mM of a mixture of amino acids (glycine, alanine, valine, leucine, isoleucine, phenylalanine, proline, tryptophan, serine, tyrosine, cysteine, methionine, asparagine, glutamine, threonine, aspartic acid, glutamic acid, lysine, arginine and histidine), 5-80 mM glucose, 1.7 mM dithiothreitol, 5-40 mM potassium phosphate, 8-20 ng/µL DNA of firefly luciferase, 0.027-0.054 mg/mL T7 RNA polymerase, 1%-4% PEG, 0.03-0.04 mM heme, 0.3-0.4 mM spermidine, and finally added 50% by volume of the yeast cell extract;
3.3 *In vitro* protein synthesis reaction: the above-mentioned reaction system was placed in an environment of 20-25 °C, and let the reaction system stand or shook slightly for a reaction for 1 to 7 hours;
3.4 Assay of the activity of luciferase: after completion of the reaction, an equal volume of luciferin as substrate was added into the wells for above reactions of a 96-well white plate or a 384-well white plate, which was immediately placed in an Envision 2120 multifunctional microplate reader (Perkin Elmer); and the absorbance was read to detect the activity of firefly luciferase, where the unit of activity is relative light unit (RLU) value.

### EXPERIMENTAL RESULTS

### 1. Examples of that biological reactions generate phosphate ion

As can be seen from Figure 1, several steps and mechanisms in biological reactions release reaction by-products represented by phosphate ion. For example, in several steps of transcription and translation, magnesium ion is involved, and the main objective product is produced through the reaction using substrates, energy, and so on in a solid state, a liquid state, or another phase state; meanwhile, in several stages of transcription and translation, several reaction by-products, such as free phosphate ion and pyrophosphate ion, are generated; for example, in kinase (protein kinase, lipid kinase, glycokinase, etc.) reaction, ATPase reaction (transmembrane transport of sodium ion, potassium ion, calcium ion, etc.) and molecular motor reaction, etc., by-products are often counteractive to biological reaction systems and inhibit the reaction. At present, known methods for removing reaction by-products mainly include dialysis method, circulation method, solution replacement method and so on; those known methods all need additional apparatus, have complex systems, are not easy to be operated and are difficult to be completed in small reaction systems. Therefore, the removal of reaction by-products, especially *in situ* removal thereof is the key to improve the reaction efficiency and to reduce the reaction complexity.

### 2. The effect of different free phosphorus control agents (agents for controlling free phosphorus) on 30 µL protein synthesis system based on cell extract

As can be seen from Figure 2, for the 30 µL protein synthesis system based on cell extract, adding too many reaction promoters would inhibit the *in vitro* protein synthesis capacity of the cell extract, and the inhibition degree of different reaction promoters against the *in vitro* protein synthesis capacity were different. When the reaction had been carried out for 3 hours, the relative light unit (RLU) value according to protein synthesis in the protein synthesis system with aluminum oxide being added at 2 hour of reaction was much (several) times higher than the RLU value according to protein synthesis in the protein synthesis system with Fe₂O₃ or Fe₃O₄ being added at the same time (the RLU value according to protein synthesis in the protein synthesis system with Fe₂O₃ or Fe₃O₄ added was very low, almost zero). In addition, the reduction of reaction activity due to aluminum oxide was relatively little; as a result, an appropriate amount of aluminum oxide as the reaction promoter of *in vitro* biological reaction has more advantages in terms of reaction effect over Fe₂O₃ and Fe₃O₄ as the reaction promoter.

### 3. The effect of different free phosphorus control agents on 120 µL protein synthesis system based on cell extract

As can be seen from Figure 3, for the 120 µL protein synthesis system based on cell extract, the *in vitro* protein synthesis capacity of cell extract would not be greatly affected or would even be slightly improved by adding appropriate amount of reaction promoter; what's more, at 2 hour of reaction, the relative light unit (RLU) value according to protein synthesis in the protein synthesis system with aluminum oxide being added was 1.4 times higher than the RLU value according to protein synthesis in the protein synthesis system with Fe₂O₃ or Fe₃O₄ being added at the same time.

### 4. Comparison of the dephosphorization effect of different dephosphorization agents in 120 µL protein synthesis system based on cell extract

As can be seen from Figure 4, when equal amount of different reaction promoters were respectively added at 2 hour of the reaction, the reaction promoters at 4.5 hour of the reaction were reduced by different degrees; relatively speaking, among them, aluminum oxide had the best control effect on the free phosphorus of the *in vitro* biological reaction system.

### 5. The effect of adding aluminum oxide to the protein synthesis system based on cell extract at different time periods

As can be seen from Figure 5, in the 90 µL cell-free reaction system, after controlling the content of free phosphorus in the system with 30 mg of aluminum oxide at 1 hour, the RLU value at 2-3 hour of reaction was 2-3 times that of the control group (without aluminum oxide). Therefore, in the 90 µL cell-free reaction system, carrying out control of free phosphorus content after reacting for 1 hour significantly improved the reaction activity of the system; what's more, when the reaction had been carried out for 3 hours, the RLU value according to protein synthesis in which aluminum oxide was added at 1 hour of reaction was significantly higher than that the RLU value according to protein synthesis in which aluminum oxide was added at 2 hour of reaction; the former was 2-3 folds of the latter.

### 6. The effect of adding aluminum oxide into the protein synthesis system based on cell extract at different time periods on the concentration of free phosphorus in the system

As can be seen from Figure 6, in the 90 µL cell-free reaction system, the concentration of free phosphorus in the system decreased significantly after reacting for 1 hour. After adding aluminum oxide at different time periods (at 1 hour and at 2 hour, respectively), the concentration of free phosphorus in the system did not rise significantly compared with that of the control group, indicating that aluminum oxide can significantly control the free phosphorus content in the system. When the reaction had been carried out for 3 hours, reduction degree of the concentration of free phosphorus in the system by addition of aluminum oxide at 1 hour of reaction and at 2 hour of reaction were relatively equal.

### 7. The effect of adding different amounts of aluminum oxide into the protein synthesis system based on cell extract after reacting for 1 hour

As can be seen from Figure 7, in the 90 µL cell-free reaction system, after adding aluminum oxide of different masses into the system at 1 hour of reaction, the RLU values were all significantly improved compared with that in the control group, wherein the RLU value could reach 2.5×10⁷. After the reaction had been carried out for 6-7 hours, the addition of 10 mg or 20 mg of aluminum oxide significantly improved the reaction activity, and the RLU value could reach 3.0×10⁷.

### 8. Phosphoric acid control system enhanced the efficacy of biological reactions.

In short, the phosphoric acid control in the system and the biochemical reaction in the system are a pair of reactions competing with each other. Excessive phosphate ion or pyrophosphate ion would cause pH imbalance in the system, affect the activity of biological enzymes in the system which participate in the biochemical reactions, and reduce the yield of objective proteins; at the same time, phosphate ion is liable to combine with magnesium ion requisite for the reaction system to form magnesium sulfate which is hardly soluble in water, leading to the reduction of reaction activity of many biochemical reaction steps because of the lack of magnesium ion. The addition of phosphoric acid control agent adjusts the balance of the biochemical reactions as described above through physical manner (adsorption) or chemical manner (forming precipitate which can be separated from the reaction system), thereby creating a relatively ideal reaction environment for the protein synthesis system.

All documents mentioned in the present invention are cited as references in this application, just as each document is individually cited as a reference. Additionally, it should be understood that those skilled in the art can make various changes or modifications to the present invention in light of the above teachings, and the equivalents also fall into the scope as defined by the appended claims of this application.

### References:

1. Garcia, R. A., & Riley, M. R. (1981). Applied biochemistry and biotechnology. Humana Press,.
2. Waddington, C. H. (1961). Molecular biology or ultrastructural biology?Nature, 190(4771), 184.
3. Assenberg, R., Wan, P. T., Geisse, S., &Mayr, L. M. (2013). Advances in recombinant protein expression for use in pharmaceutical research. Current Opinion in Structural Biology, 23(3), 393 - 402.
4. Gräslund, S., Nordlund, P., Weigelt, J., Hallberg, B. M., Bray, J., Gileadi, O., ···Gunsalus, K. C. (2008). Protein production and purification. Nature Methods, 5(2), 135-146.
5. Katzen, F., Chang, G, &Kudlicki, W. (2005). The past, present and future of cell-free protein synthesis. Trends in Biotechnology, 23(3), 150-156.
6. Mcmahon, T., Zijl, P. C. M. Van, & Gilad, A. A. (2015). NIH Public Access, 27(3), 320-331.
7. Quast, R. B., Ballion, B., Stech, M., Sonnabend, A., Varga, B. R., Wüstenhagen, D. A., ···Kubick, S. (2016). Cell-free synthesis of functional human epidermal growth factor receptor: Investigation of ligand-independent dimerization in Sf21 microsomal membranes using non-canonical amino acids. Scientific Reports, 6(March), 1-13.
8. Lu, Y. (2017). Cell-free synthetic biology: Engineering in an open world. Synthetic and Systems Biotechnology, 2(1), 23-27.
9. Robertis, D. (1987). Cell and molecular biology. 8th Edition.
10. Fromm, H. J., & Hargrove, M. (2012). Essentials of Biochemistry.

## Claims

1. An *in vitro* protein synthesis system, comprising:
(a) cells, cell extract, or a combination thereof;
(b) a first reaction promoter, and the first reaction promoter is selected from the group consisting of aluminum, aluminum salt, oxy-aluminum composite, and combinations thereof.

2. The *in vitro* protein synthesis system of claim 1, wherein the cells are selected from the group consisting of *E. coli,* bacteria, mammalian cells (e.g., HF9 cells, Hela cells, CHO cells, HEK293 cells), plant cells, yeast cells, and combinations thereof.

3. An *in vitro* cell-free protein synthesis system, comprising:
(a) cell extract; and
(b) a first reaction promoter, and the first reaction promoter is selected from the group consisting of aluminum, aluminum salt, oxy-aluminum composite, and combinations thereof.

4. The protein synthesis system of claim 1 or claim 3, wherein the oxy-aluminum composite comprises oxy-aluminum nanoparticles.

5. The protein synthesis system of claim 1 or claim 3, wherein the oxy-aluminum composite comprises Al₂O₃.

6. The protein synthesis system of claim 1 or claim 3, wherein particle size of the oxy-aluminum composite is in a range of 0.5-20 mm, preferably, in a range of 0.8-10 mm, more preferably, in a range of 1-5 mm.

7. The protein synthesis system of claim 1 or claim 3, wherein average weight of the oxy-aluminum composite is in a range of 1-80 mg, preferably, in a range of 3-50 mg, more preferably, in a range of 4-30 mg.

8. The protein synthesis system of claim 1 or claim 3, wherein in the first reaction promotor, the content (wt%) of the oxy-aluminum composite is in a range of 0.1%-20%, preferably, in a range of 1%-10%, more preferably, in a range of 2%-8%, more preferably, in a range of 4%-7%, more preferably, in a range of 5.5%-6.5%, based on the total weight of the first reaction promoter.

9. The protein synthesis system of claim 1 or claim 3, wherein in the protein synthesis system, the content (wt%) of the first reaction promoter is in a range of 20%-70%, preferably, in a range of 30%-60%, more preferably, in a range of 45%-55%, based on the total weight of the protein synthesis system.

10. A method for producing the protein synthesis system of claim 1 or claim 3, comprising the steps of:
mixing component (i) and component (ii) to obtain the protein synthesis system of claim 1 or claim 3, wherein the component (i) is selected from the group consisting of cells, cell extract and a combination thereof; the component (ii) is a first reaction promoter, and the first reaction promoter is selected from the group consisting of aluminum, aluminum salt, oxy-aluminum composite, and combinations thereof.

11. The method of claim 10, wherein in the protein synthesis system, the mass ratio of the component (i) to the component (ii) is 0.1-10: 0.1-10, preferably, 0.5-8: 0.5-8, more preferably, 0.8-5: 0.8-5, more preferably, 0.9-2: 0.9-2.

12. A method for *in vitro* protein synthesis, comprising steps of:
(i) providing the protein synthesis system of claim 1 or claim 3, and adding exogenous DNA molecules for guiding protein synthesis; and
(ii) incubating the protein synthesis system provided in the step (i) for a period of time T1 under suitable conditions to synthesize protein encoded by the exogenous DNA.

13. The method of claim 12, wherein the method further comprises: (iii) optionally, isolating the protein encoded by the exogenous DNA from the protein synthesis system, or
detecting the protein encoded by the exogenous DNA from the protein synthesis system.

14. A kit, comprising:
(k1) a first container, and component (i) contained in the first container, wherein the component (i) is selected from the group consisting of cells, cell extract, and a combination thereof;
(k2) a second container, and component (ii) contained in the second container, wherein the component (ii) is a first reaction promoter, and the first reaction promoter is selected from the group consisting of aluminum, aluminum salt, oxy-aluminum composite, and combinations thereof; and
(kt) a label or an instruction manual.
